# FASCICULE DE BREVET EUROPEEN

(11) **EP 4 618 921 B1**
(45) Date de publication et mention de la délivrance du brevet: **10.06.2026**
(21) Numéro de dépôt: 22826672.2
(22) Date de dépôt: 15.11.2022
(51) Int. Cl.: A61F 13/08, A41B 11/00, A41B 11/08, A41D 13/12, A61F 13/14

(54) **VETEMENT DE COMPRESSION POST-OPERATOIRE**
POSTOPERATIVES KOMPRESSIONSKLEIDUNGSSTÜCK
POST-OPERATIVE COMPRESSION GARMENT

(43) Date de publication de la demande: 24.09.2025
(73) Titulaire: Medical Z, 37550 Saint Avertin (FR)
(72) Inventeur: TEDGUI ZAGAME, Yann, HOUSTON TEXAS, 77077 (US); MOJALLAL, Alain Ali, 69006 LYON (FR)
(74) Mandataire: Regimbeau
(86) Numéro de dépôt international: PCT/FR2022/000111
(87) Numéro de publication internationale: WO 2024/105309

(56) Documents cités:
- EP-A1- 2 881 094
- EP-B1- 3 493 690
- US-A1- 2014 230 124
- US-A1- 2015 190 669
- US-A1- 2017 245 556
- US-B2- 10 596 764

## Description

### DOMAINE DE L'INVENTION

La présente invention concerne un vêtement de compression destiné à une utilisation post-opératoire.

### ETAT DE LA TECHNIQUE

A la suite d'une intervention chirurgicale, l'inflammation et la formation d'œdèmes et d'hématomes sont très courantes. Ces œdèmes et hématomes sont douloureux et peuvent gêner la bonne cicatrisation du tissu. Il y également un risque de formation de caillots de sang. Dans certains cas, ces phénomènes peuvent prendre jusqu'à plusieurs semaines avant de disparaître complètement.

L'application d'une compression sur la zone d'œdèmes et d'hématomes permet de diminuer ces effets indésirables. Cependant, l'application d'une compression sur le corps d'un patient est délicate à mettre en œuvre, notamment car chaque opération est faite de décollements, dissections, infiltrations, œdèmes et cicatrices différents et nécessite une compression adaptée, tout en protégeant des zones de cicatrices spécifiques à l'intervention.

Après une intervention de chirurgie plastique, il peut y avoir en outre un risque de relâchement de la peau qui pourrait entrainer un résultat disgracieux. Ce risque peut être limité en portant un vêtement compressif tel que divulgué par exemple dans le document US10596764. maintenant les tissus en place et aidant au redrapage cutané.

Les besoins en compression évoluent dans le temps suivant l'intervention en fonction des phases de la cicatrisation et de la maturation post-opératoire. Par exemple, certaines zones peuvent être très fragiles en phase post-opératoire immédiate, alors qu'un traitement compressif peut être bénéfique dans une phase ultérieure.

### EXPOSE DE L'INVENTION

Un but de l'invention est de concevoir un vêtement de compression destiné à une utilisation post-opératoire qui procure une amélioration de la guérison de la zone anatomique traitée.

A cette fin, l'invention propose un vêtement de compression pour utilisation post-opératoire adapté pour assurer une compression d'une partie du corps d'un patient, caractérisé en ce qu'il comprend :
- au moins un textile compressif, et
- une pluralité de pistes de silicone disposées sur une pluralité de bandes de support agencées sur une face du textile compressif destinée à être en contact avec la peau du patient, lesdites bandes de support présentant un point commun dans une portion supérieure du vêtement, la distance entre les bandes de support augmentant à une distance croissante du point commun de sorte que les pistes de silicone adhèrent à la peau du patient suivant une voie de drainage lymphatique respective de ladite partie du corps du patient.

Un tel vêtement de compression permet une conception par zone du corps du patient et par type de chirurgie. Les pistes en silicone permettent un auto-drainage lymphatique sur les voies lymphatiques. En outre, le vêtement permet une conception modulable en fonction de la période post-opératoire.

Le port d'un tel vêtement de compression permet de diminuer les œdèmes, les ecchymoses et les douleurs chez le patient et d'accélérer et améliorer la cicatrisation. Il permet en outre d'améliorer le confort du patient et d'accélérer la reprise d'une activité physique après l'intervention chirurgicale. Par ailleurs, le port du vêtement de compression améliore le remodelage cutané et le résultat de l'opération.

De manière avantageuse, le textile compressif comprend au moins deux zones assemblées par des coutures élastiques dont le surplus de couture est agencé à l'extérieur du vêtement. Ainsi, les différents gradients de compression peuvent être appliqués en fonction du type de chirurgie.

De préférence, les pistes de silicone forment un relief sur la bande de support. Avantageusement, les pistes de silicone sont ondulées, de préférence sinusoïdales.

Avantageusement, chaque bande de support comporte plusieurs pistes de silicone parallèles, de préférence deux pistes de silicone parallèles.

Dans certains modes de réalisation, le vêtement de compression se présente sous la forme d'une gaine de compression abdominale

Dans d'autres modes de réalisation, le vêtement de compression se présente sous la forme d'une gaine de compression des membres inférieurs.

Dans d'autres modes de réalisation, le vêtement de compression se présente sous la forme d'une gaine de compression mammaire

Un autre objet de l'invention concerne un ensemble comprenant un vêtement de compression et au moins une plaque auto-adhésive apte à être insérée entre ledit vêtement de compression et une zone du corps du patient.

Un autre objet de l'invention concerne un ensemble comprenant un vêtement de compression selon l'une des revendications précédentes et au moins un coussin de compression en mousse apte à être inséré entre ledit vêtement de compression et une zone du corps du patient.

L'invention se rapporte aussi à un procédé de fabrication d'un vêtement de compression d'une partie du corps d'un patient, comprenant
- la fourniture d'au moins deux de bandes de support comportant au moins une piste de silicone respective, et
- l'agencement desdites au moins deux bandes de support sur une face intérieure d'un vêtement en un textile compressif, de sorte que les pistes de silicone adhèrent à la peau du patient, les bandes de support présentant un point commun dans une portion supérieure du vêtement, la distance entre les bandes de support augmentant à une distance croissante du point commun suivant au moins une voie de drainage lymphatique de ladite partie du corps du patient.

### BREVE DESCRIPTION DES FIGURES

D'autres caractéristiques et avantages de l'invention ressortiront de la description détaillée qui va suivre, en référence aux dessins annexés, sur lesquels :
La figure 1A est une vue schématique d'une gaine de compression abdominale destinée à être portée après une lipoaspiration abdominale.
La figure 1B est une vue schématique d'une gaine de compression abdominale destinée à être portée après une dermolipectomie abdominale avec lipoaspiration avec incision basse et décollement central.
La figure 1C est une vue latérale d'une gaine abdominale portée par un patient.
La figure 1D est une vue de dos d'une gaine abdominale portée par un patient.
La figure 2A est une vue schématique de la face avant d'une gaine de compression des membres inférieurs.
La figure 2B est une vue schématique de dos d'une gaine de compression des membres inférieurs.
La figure 2C est une vue latérale d'une gaine de compression des membres inférieurs portée par un patient.
La figure 2D est une vue de dos d'une gaine de compression des membres inférieurs portée par un patient.
La figure 2E illustre une zone de tissu comportant des bandes de support comportant des pistes de silicone, destinée à former une portion d'une gaine de compression des membres inférieurs.
La figure 3 est une vue schématique d'un vêtement de compression mammaire destiné à être porté après une intervention chirurgicale des seins.
La figure 4 illustre une bande de silicone apte à être agencée selon les voies anatomiques de drainage lymphatique.
La figure 5 illustre une plaque auto-adhésive apte à être utilisée ensemble avec un vêtement de compression.
La figure 6 illustre un coussin de compression en mousse apte à être utilisé ensemble avec un vêtement de compression.

### DESCRIPTION DETAILLEE DE MODES DE REALISATION

L'invention propose un vêtement de compression destiné à être porté typiquement après une intervention de chirurgie plastique et esthétique induisant un oedème et/ou une cicatrice au niveau d'une partie du corps d'un patient. Néanmoins, l'utilisation d'un tel vêtement n'est pas limitée à la chirurgie plastique mais peut également être envisagée après un autre type d'intervention chirurgicale. A titre d'exemple illustratif et non limitatif, le vêtement peut être destiné à être porté après une intervention de chirurgie digestive avec laparotomie médiane, de chirurgie orthopédique avec les incisions de genou ou de hanche et une intervention chirurgicale des seins.

Le vêtement est conçu en fonction de la zone anatomique concernée et en fonction de l'intervention chirurgicale réalisée sur le patient. Par exemple, une gaine de compression abdominale peut être conçue pour une lipoaspiration abdominale seule sans incision et sans décollement ou pour une lipo-abdominoplastie avec incision basse et décollement central, ou pour une laparotomie verticale médiane. Une gaine des membres inférieurs peut être conçue pour une lipoaspiration des membres inférieurs seuls ou une dermolipectomie des cuisses (cruroplastie) avec lipoaspiration avec incision médiane ou postéro-médiane. Un vêtement de compression mammaire peut être conçu pour une réduction mammaire avec mastopexie ou pour une augmentation mammaire par prothèses seules ou par technique composite comportant une prothèse et une injection de graisse autologue.

Le vêtement de compression comprend au moins une zone de textile présentant une extensibilité choisie en fonction de la compression à exercer sur la partie du corps du patient couverte par ladite zone. Chaque textile est typiquement constitué d'un tissu chaîne et trame, qui est extensible en chaîne et en trame, c'est-à-dire que ce tissu est à même de s'allonger élastiquement suivant ses fils de chaîne et suivant ses fils de trame.

Chaque fil de chaîne et de trame peut comprendre dans sa composition : des fibres naturelles, telles que du coton ou du lin des fibres synthétiques, telles que du polyamide, du polyester, du Nylon, de l'élasthanne ou Lycra^{™}, ou un mélange de plusieurs des fibres précitées.

Un textile de ce type présente un allongement maximal en chaîne et en trame, c'est-à-dire un allongement à la rupture dans la direction de ses fils de chaîne et de trame. Le(s) tissus constituant le vêtement compressif présente(nt) typiquement une extensibilité sensiblement homogène en chaîne et en trame. Dans d'autres modes de réalisation, d'autres structures de textile et d'autres extensibilités peuvent être utilisées.

Lorsque le vêtement de compression est porté par le patient, chaque zone de textile exerce sur le corps du patient une compression dont l'intensité dépend de l'extensibilité du textile. Une forte extensibilité du textile a pour effet une faible compression sur le corps du patient, et une faible extensibilité du textile produit une forte compression sur le corps du patient. L'extensibilité de chaque textile peut donc être ajustée à l'effet recherché, à savoir exercer une compression suffisante pour faciliter ou accélérer la réduction d'œdèmes et d'hématomes post-opératoires tout en exerçant une pression moins forte sur les zones fragilisées par l'intervention chirurgicale, en permettant les mouvements du patient. Généralement, la compression est comprise entre 17 et 24 mm Hg. Eventuellement, plusieurs zones de tissus présentant des extensibilités différentes peuvent être assemblées dans un vêtement.

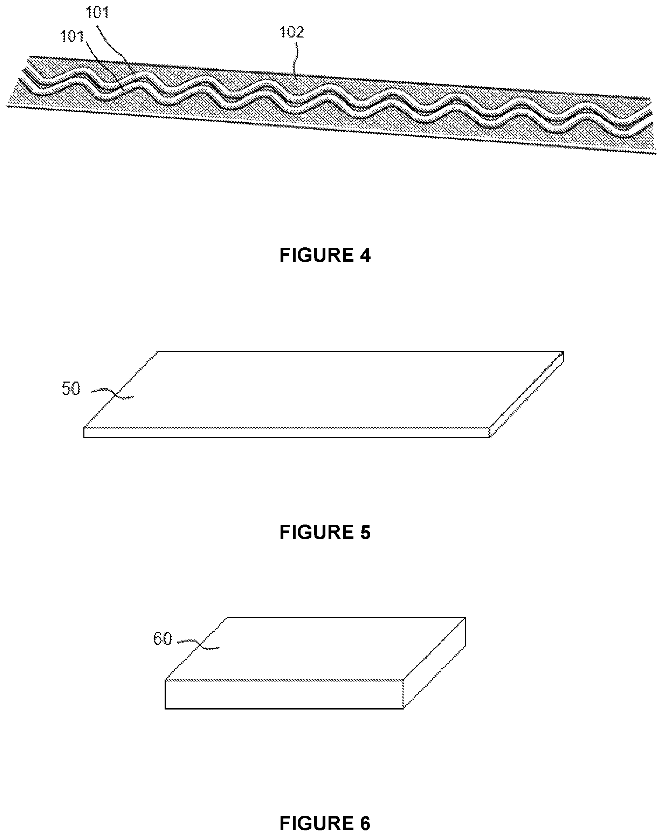

Le vêtement de compression comporte par ailleurs des pistes de silicone 1 agencées sur la surface intérieure du vêtement. Chaque piste de silicone est portée par une bande de support en textile élastique fixée sur l'intérieur du vêtement. Lesdites pistes de silicone 1 sont orientées selon les voies anatomiques de drainage lymphatique, qui sont connues de l'homme du métier. Les pistes de silicone sont par exemple agencées en zigzag ou selon un motif sinusoïdal et peuvent présenter un relief, par exemple d'une hauteur de 2 mm. En pratique, une ou plusieurs pistes de silicone peuvent être agencées sur une face d'une bande de support sous forme d'un ruban en textile, dont la face opposée peut être appliquée sur la face intérieure du vêtement et fixée par couture ou collage. La figure 4 illustre une telle bande de support sous forme d'un ruban 102 en textile. La bande de support comporte deux pistes en relief 101 en silicone. Avantageusement, les pistes sont ondulées, de préférence sinusoïdales. Lorsque le vêtement est porté, les pistes de silicone 1 sont appliquées contre la peau du patient. Le silicone procure une certaine adhésion des pistes de silicone 1 sur la peau. Ainsi, lorsque que le patient se mobilise, l'effet de micro-mouvement et micro-cisaillement généré par ces pistes de silicone adhérant à la peau joue le rôle d'un drainage lymphatique guidé orientant la lymphe vers les aires ganglionnaires. De plus, grâce à ces pistes de silicone qui adhèrent à la peau, le vêtement compressif est mieux stabilisé sur la peau, notamment dans les zones difficiles à faire tenir comme, par exemple dans le cas d'une gaine de compression abdominale, les racines des cuisses ou la région sous costale.

Dans un mode de réalisation particulièrement avantageux, plusieurs pistes de silicone sont agencées ensembles sur une bande de support qui est maintenue sur le tissu par une ou plusieurs coutures. De préférence, deux pistes parallèles en silicone sont en contact avec la peau du patient lorsque le vêtement est porté. Les deux pistes présentent un motif sinusoïdal en relief. La présence de deux pistes parallèles permet un maintien du vêtement sur la peau du vêtement amélioré par rapport à l'utilisation d'une seule piste.

L'agencement des pistes de silicone et des bandes de support est tel que plusieurs pistes de silicone se rejoignent à un point commun dans une zone supérieure du vêtement. Dans le cas d'un pantalon ou une gaine, le point commun est situé au niveau de la taille du patient. Les bandes de support comportant les pistes de silicone rayonnent vers le bas à partir de ce point commun le long des voies lymphatiques du patient, de sorte que la distance entre les bandes de support respectives augmente à une distance croissante du point commun, en direction du bas du vêtement.

De manière avantageuse, les zones de textile constituant le vêtement de compression sont assemblées entre elles par des coutures élastiques dont le surplus de couture est agencé de préférence à l'extérieur du vêtement. Lesdites coutures élastiques sont préférablement agencées de manière à ne pas couvrir la zone d'incision et la zone du décollement du corps du patient lorsque le vêtement est porté par le patient.

On va maintenant décrire différents modes de réalisation d'un tel vêtement de compression.

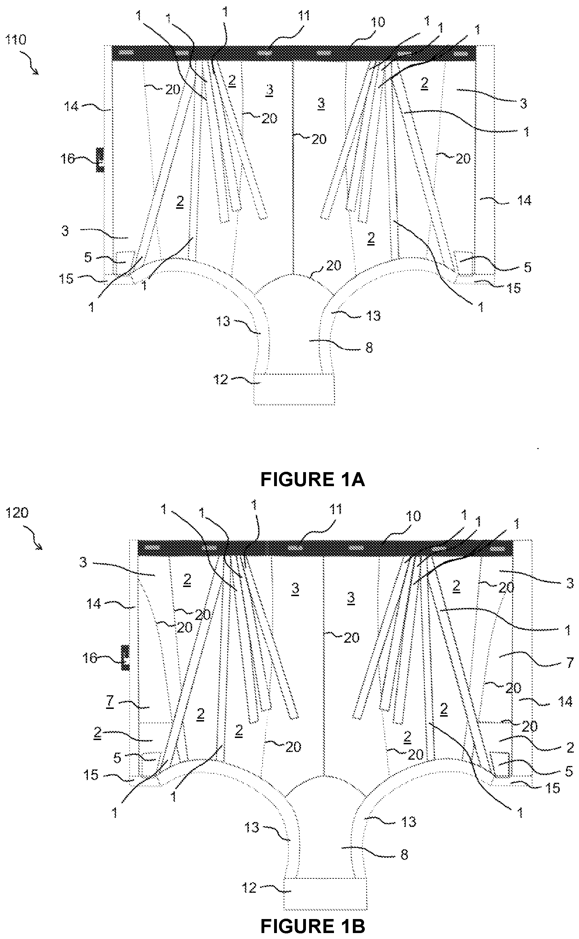

La figure 1A est une vue schématique d'un premier mode de réalisation d'un vêtement de compression postopératoire, à savoir une première gaine de compression abdominale 110. La gaine 110 est conçue pour être portée par un patient après une intervention chirurgicale au niveau de l'abdomen du patient, et en particulier après une intervention de liposuccion abdominale.

La gaine de compression abdominale 110 comporte plusieurs zones d'un premier textile 2 et plusieurs zones d'un deuxième textile 3. Le premier textile 2 présente une extensibilité inférieure à celle du deuxième textile 3. Les différentes zones de textile sont avantageusement assemblées par des coutures élastiques 20. Par ailleurs, la gaine comprend plusieurs bandes de silicone 1 agencées suivant des voies de drainage lymphatique de la région abdominale.

Lorsque la gaine est portée par le patient, une première zone comportant le premier textile 2 est agencée de sorte à exercer une première compression imposant une forme concave dans des régions postérieures et latérales du patient, et la deuxième zone comportant le deuxième textile 3 est agencée de sorte à exercer une deuxième compression homogène et inférieure à la première compression exercée sur une région abdominale du patient.

En combinaison avec ces compressions, les pistes de silicone 1 assurent un drainage lymphatique de la région abdominale.

Sur le bord supérieur, la gaine 110 comporte une bande élastique 10 comprenant des passants 11 aptes à fixer des bretelles (non représentées). En outre, la gaine 110 comporte une bande de porte 14 permettant de fermer la gaine au niveau du ventre du patient par des crochets 16. Un biais élastique 15 est agencé au bord inférieur de la zone de fermeture.

Ladite gaine 110 comporte un fond 8 en coton apte à être fermé et ouvert par une bande de velcro doux 12 qui est conçue pour être accroché aux bandes de velcro agrippantes 5, ledit fond 8 permettant de maintenir la gaine en place quand elle est portée par le patient. Des bandes de dentelle 13 peuvent être agencées sur les ouvertures permettant le passage des cuisses.

Sur la figure 1B est représenté un deuxième mode de réalisation d'un vêtement de compression postopératoire, à savoir une deuxième gaine de compression abdominale 120.

Les éléments désignés par les mêmes signes de référence que sur la figure 1A sont identiques ou remplissent la même fonction que les éléments déjà décrits dans le premier mode de réalisation et ne seront donc pas décrits à nouveau.

La gaine 120 est conçue pour être portée par un patient après une intervention chirurgicale au niveau de l'abdomen du patient, et en particulier après une intervention d'abdominoplastie comme par exemple une dermolipectomie abdominale avec lipoaspiration avec incision basse et décollement central.

La gaine de compression abdominale 120 comporte plusieurs zones d'un premier textile 2, plusieurs zones d'un deuxième textile 3 et une ou plusieurs zones d'un troisième textile 7. Le premier textile 2 présente une extensibilité inférieure à celle du deuxième textile 3, qui est elle-même inférieure à celle du troisième textile 7. Les différentes zones de textile sont avantageusement assemblées par des coutures élastiques.

Une première zone comportant le premier textile 2 est agencée de sorte à exercer une première compression imposant une forme concave dans des régions postérieures et latérales du patient, et la deuxième zone comportant le deuxième textile 3 est agencée de sorte à exercer une deuxième compression homogène et inférieure à la première compression exercée sur une partie de la région abdominale du patient, et la troisième zone comportant le textile 7 exerce une pression très faible sur la zone antérieure et centrale qui correspond à la zone de décollement.

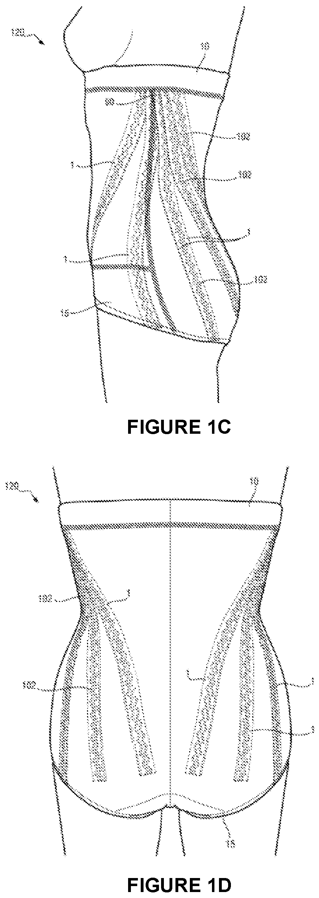

Les figures 1C et 1D représentent une gaine de compression abdominale 120 portée par un patient. Les bandes de support 102 comportant les pistes de silicone 1 indiquées en pointillées sont agencées sur la face intérieure de la gaine en contact avec la peau du patient.

Les bandes de support comportant des pistes de silicone se rejoignent à un point commun 90 à proximité du bord supérieur de la gaine comportant une bande élastique 10. La distance entre les bandes de support comportant des pistes de silicone augmente vers la partie inférieure de la gaine et est maximale au niveau du bord inférieur comportant un biais élastique 15.

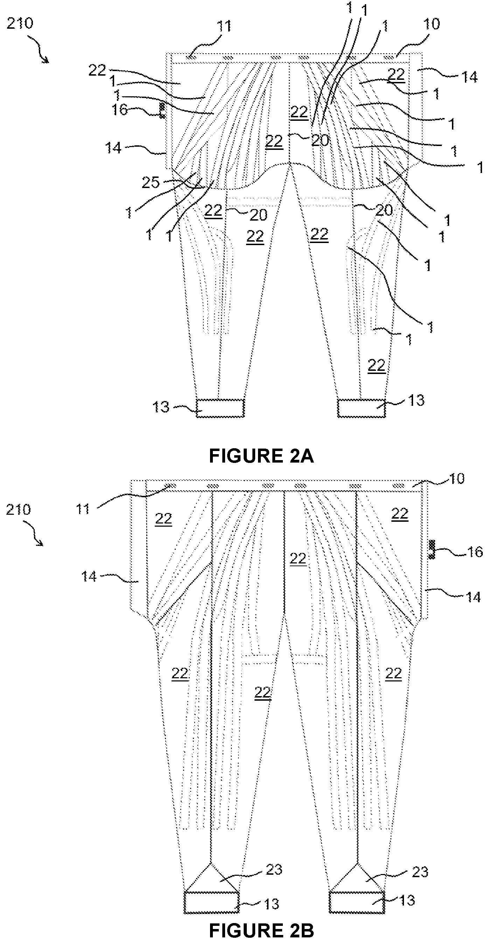

Les figures 2A et 2B présentent une gaine de compression des membres inférieurs 210 conçue pour être portée après une lipoaspiration des cuisses ou des fesses. La figure 2A présente une vue schématique de la face de devant de ladite gaine 210, la figure 2B présente une vue schématique de la vue de dos de ladite gaine 210.

La gaine de compression des membres inférieurs 210 comporte plusieurs zones d'un textile 22 assemblées entre elles par des coutures élastiques 20. Par ailleurs, la gaine comprend plusieurs pistes de silicone 1 agencées suivant des voies de drainage lymphatique des membres inférieurs. Sur le bord supérieur, la gaine de compression des membres inférieurs 210 comporte une bande élastique 10 comprenant des passants 11 aptes à fixer des bretelles (non représentées). En outre, la gaine 210 comporte deux bandes de porte 14 qui permettent de fermer le corsaire sur les côtés par des crochets 16.

Ladite gaine 210 peut comporter une ouverture au niveau du pubis (non représentée) ou, de manière alternative, comporter un fond fermé

La gaine 210 peut comporter en outre un biais élastique 25 traversant sur la largeur de la face avant du corsaire agencé au niveau du pubis, agencée pour former une bordure pour la portion du tissu constituant le bord de l'ouverture sur la face avant le cas échéant. Des bandes de dentelle 13 peuvent être agencées sur les ouvertures inférieures permettant le passage des pieds. Au niveau des mollets, des pièces 23 d'un tissu présentant une forte extensibilité peuvent être agencées, exerçant une pression très faible et augmentant ainsi le confort du patient portant la gaine 210 des membres inférieurs.

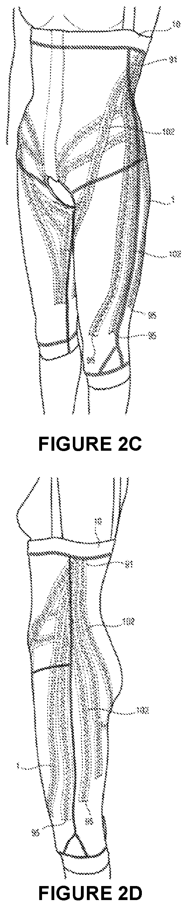

Les figures 2C et 2D représentent une gaine de compression des membres inférieurs portée par un patient. Les bandes de support 102 comportant les pistes de silicone 1 indiquées en pointillées sont agencées sur la face intérieure de la gaine en contact avec la peau du patient.

Les pistes de silicone se rejoignent à un point commun 91 en proximité du bord supérieur de la gaine comportant une bande élastique 10. La distance entre les pistes de silicone augmente vers la partie inférieure de la gaine et est maximale au niveau de leur extrémité inférieure 95 au niveau du genou du patient.

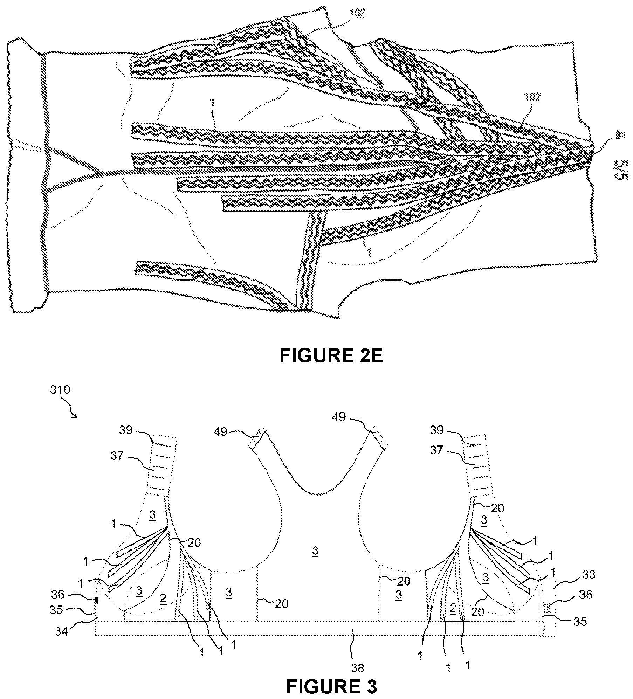

La figure 2E illustre une zone de tissu découpé pour former une portion de la gaine de compression des membres inférieurs. Les bandes de support comportant les pistes de silicone sont cousues sur la zone de tissu. Plusieurs bandes de support comportant des pistes de silicone se joignent à un point commun destiné à former le bord supérieur du vêtement sur lequel une bande élastique sera fixée. D'autres bandes de support comportant des pistes de silicone destinées à appliquer une compression sur d'autres voies de drainage lymphatique sont agencées sur la zone de tissu.

La figure 3 présente un vêtement de compression mammaire 310 conçu pour être porté après une intervention chirurgicale des seins. Une telle intervention est par exemple une réduction mammaire avec mastopexie ou une augmentation mammaire, par exemple par prothèses seules ou par une technique composite comportant une prothèse et une injection de graisse autologue.

Le vêtement de compression mammaire 310 comporte plusieurs zones d'un premier textile 2 et plusieurs zones d'un deuxième textile 3. Le premier textile 2 présente une extensibilité inférieure à celle du deuxième textile 3. Par ailleurs, le vêtement 310 comprend plusieurs bandes de support comportant des pistes de silicone 1 agencées suivant des voies de drainage lymphatique de chaque sein.

Lorsque le vêtement 310 est porté par le patient, une première zone comportant le premier textile 2 est agencée de sorte à exercer une première compression entre la partie latérale du sein et la partie latéro-thoracique, permettant une mise en forme du sein à partir du sillon latéro-mammaire. La deuxième zone comportant le deuxième textile 3 est agencée de sorte à exercer une deuxième compression inférieure à la première compression sur les parties latérales et la partie centrale du dos. En combinaison avec ces compressions, les pistes de silicone 1 assurent un drainage lymphatique des seins.

Dans une zone centrale du dos et sur une partie de chaque sein, le vêtement comporte en outre une doublure thermorégulatrice, agencé en-dessous d'une zone du deuxième textile 3. Ladite doublure thermorégulatrice permet de réduire la transpiration du patient.

Le vêtement de compression mammaire comporte une ceinture élastique 38 agencé au-dessous du niveau des seins, apte à maintenir le vêtement en place. Une fermeture à glissière divisible 35 et des crochets 36 sont agencés sur les bords antérieurs du vêtement, permettant la fermeture dans une zone centrale de la poitrine du patient. Le vêtement peut comporter une pièce 33 en mousse dans la zone centrale de la poitrine, augmentant le confort du patient. En outre, un ou plusieurs rubans 34 peuvent être agencés dans ladite zone formant une bordure sur les bords antérieurs du vêtement.

Deux bandes 37 sont agencées dans la partie au-dessus du sein et passant sur les épaules du patient, lesdites bandes 37 comprenant chacune plusieurs attaches 39 aptes à être fixées sur des boucles 49 agencées des deux côtés de la partie centrale du dos du vêtement, lesdites plusieurs attaches permettant d'adapter longueur de la bande 37 nécessaire pour le maintien du sein en choisissant l'attache 39 la plus adaptée à la morphologie du patient.

L'invention n'est pas limitée aux exemples de vêtement décrits mais est applicable à une multitude de vêtements de compression postopératoire à drainage lymphatique, en appliquant à ces derniers les considérations techniques développées jusqu'ici. D'autres modes de réalisation peuvent comprendre, à titre illustratif et non limitatif, des vêtements de compression couvrant les bras, le dos, ou un seul membre supérieur ou inférieur.

Le vêtement de compression peut être utilisé ensemble avec d'autres éléments qui peuvent être ajoutés dans des phases ultérieures de guérison suivant l'intervention chirurgicale.

Par exemple, dans une première phase de guérison, il peut être préférable de ne pas exercer une compression trop importante sur les tissus opérés.

Lorsque l'intervention chirurgicale a généré une cicatrice, il peut être utile, dans une deuxième phase dans laquelle la cicatrice est refermée, d'appliquer sur la cicatrice un élément adapté pour masser la cicatrice afin de l'estomper.

Par ailleurs, un ou plusieurs coussins de compression 60 en mousse, comme illustrés sur la figure 6, peuvent être agencés entre le vêtement de compression et le corps du patient, pour augmenter la compression dans une zone initialement peu comprimée. Par exemple, de tels coussins peuvent être placés dans la partie antérieure et sous-ombilicale qui correspond à la zone du décollement quand le vêtement est une gaine de compression abdominale. De tels coussins de compression 60 en mousse sont typiquement ajoutés environ après 5-7 jours suivant l'intervention chirurgicale. Une compression avant ce délai peut diminuer la perfusion et aggraver le risque de nécrose cutanée. Les coussins ont pour objectif de comprimer fortement pour modeler et bien plaquer la peau afin d'améliorer le résultat esthétique de l'intervention chirurgicale. Bien que le coussin 60 soit représenté avec une forme parallélépipédique sur la figure 6, il va de soi que toute autre forme, notamment adaptée à la morphologie du patient, peut être employée.

Par ailleurs, dans certaines situations, une ou plusieurs plaques auto-adhésives 50, telles qu'illustrées sur la figure 5, peuvent être insérées entre le vêtement de compression et le corps du patient, au niveau de la cicatrice en cours de guérison. Par exemple, de telles plaques 50 peuvent être placées au niveau du pli sous-pubien quand le vêtement est une gaine de compression abdominale utilisée après une plastie abdominale. Ces plaques 50 sont d'abord collées sur la peau du patient puis sont maintenues en place par la pression appliquée par ledit vêtement de compression. De telles plaques 50 sont typiquement insérées après la cicatrisation de l'incision, dont la durée est typiquement entre 3 et 4 semaines. Lesdites plaques, qui sont conformables au corps du patient, peuvent être constituées de silicone ou d'un mélange de copolymère tri-bloc et d'huile minérale. Bien que la plaque 50 soit représentée avec une forme parallélépipédique sur la figure 5, il va de soi que toute autre forme, notamment adaptée à la morphologie du patient, peut être employée.

## Revendications

1. Vêtement de compression pour utilisation post-opératoire adapté pour assurer une compression d'une partie du corps d'un patient, **caractérisé en ce qu'**il comprend :
o au moins un textile compressif comprenant au moins deux zones assemblées par des coutures élastiques (20) dont le surplus de couture est agencé à l'extérieur du vêtement, et
o une pluralité de pistes de silicone (1) disposées sur une pluralité de bandes de support agencées sur une face du textile compressif destinée à être en contact avec la peau du patient, lesdites bandes de support présentant un point commun dans une portion supérieure du vêtement, la distance entre les bandes de support augmentant à une distance croissante du point commun de sorte que les pistes de silicone adhèrent à la peau du patient suivant une voie de drainage lymphatique respective de ladite partie du corps du patient.

2. Vêtement de compression selon la revendication 1, dans lequel les pistes de silicone (1) forment un relief sur la bande de support.

3. Vêtement de compression selon l'une des revendications précédentes dans lequel les pistes de silicone (1) sont ondulées, de préférence sinusoïdales.

4. Vêtement de compression selon l'une des revendications précédentes, dans lequel chaque bande de support comporte plusieurs pistes de silicone parallèles, de préférence deux pistes de silicone parallèles.

5. Vêtement de compression selon l'une des revendications précédentes, se présentant sous la forme d'une gaine de compression abdominale.

6. Vêtement de compression selon l'une des revendications 1 à 5, se présentant sous la forme d'une gaine de compression des membres inférieurs (210).

7. Vêtement de compression selon l'une des revendications 1 à 5, se présentant sous la forme d'une gaine de compression mammaire (310).

8. Ensemble comprenant un vêtement de compression selon l'une des revendications précédentes et au moins une plaque (50) auto-adhésive apte à être insérée entre ledit vêtement de compression et une zone du corps du patient.

9. Ensemble comprenant un vêtement de compression selon l'une des revendications précédentes et au moins un coussin de compression (60) en mousse apte à être inséré entre ledit vêtement de compression et une zone du corps du patient.

10. Procédé de fabrication d'un vêtement de compression d'une partie du corps d'un patient, comprenant :
la fourniture d'au moins deux bandes de support comportant au moins une piste de silicone respective (1), et
l'agencement desdites au moins deux bandes de support sur une face intérieure d'un vêtement en un textile compressif comprenant au moins deux zones assemblées par des coutures élastiques (20) dont le surplus de couture est agencé à l'extérieur du vêtement, de sorte que les pistes de silicone soient orientées du côté de la peau du patient, les bandes de support présentant un point commun dans une portion supérieure du vêtement, la distance entre les bandes de support augmentant à une distance croissante du point commun suivant au moins une voie de drainage lymphatique de ladite partie du corps du patient.

## Patentansprüche

1. Kompressionskleidungsstück zur postoperativen Verwendung, geeignet, eine Kompression eines Körperteils eines Patienten sicherzustellen, **dadurch gekennzeichnet, dass** es umfasst :
• mindestens einen kompressiven Textilstoff, der mindestens zwei Zonen umfasst, die durch elastische Nähte (20) zusammengefügt sind, wobei der Nahtüberschuss an der Außenseite des Kleidungsstücks angeordnet ist, und
• eine Vielzahl von Silikonbahnen (1), die auf einer Vielzahl von Trägerbändern angeordnet sind, welche auf einer Seite des kompressiven Textilstoffs angeordnet sind, die dazu bestimmt ist, mit der Haut des Patienten in Kontakt zu kommen, wobei die genannten Trägerbänder einen gemeinsamen Punkt in einem oberen Abschnitt des Kleidungsstücks aufweisen, und der Abstand zwischen den Trägerbändern mit zunehmender Entfernung vom gemeinsamen Punkt zunimmt, so dass die Silikonbahnen an der Haut des Patienten entlang eines jeweiligen Lymphdrainagewegs des genannten Körperteils des Patienten haften.

2. Kompressionskleidungsstück nach Anspruch 1, bei dem die Silikonbahnen (1) eine Erhebung auf dem Trägerband bilden.

3. Kompressionskleidungsstück nach einem der vorhergehenden Ansprüche, bei dem die Silikonbahnen (1) gewellt, vorzugsweise sinusförmig sind.

4. Kompressionskleidungsstück nach einem der vorhergehenden Ansprüche, bei dem jedes Trägerband mehrere parallele Silikonbahnen aufweist, vorzugsweise zwei parallele Silikonbahnen.

5. Kompressionskleidungsstück nach einem der vorhergehenden Ansprüche, das in Form einer abdominalen Kompressionsbandage vorliegt.

6. Kompressionskleidungsstück nach einem der Ansprüche 1 bis 5, das in Form einer Kompressionsbandage der unteren Gliedmaßen (210) vorliegt.

7. Kompressionskleidungsstück nach einem der Ansprüche 1 bis 5, das in Form einer Brustkompressionsbandage (310) vorliegt.

8. Satz umfassend ein Kompressionskleidungsstück nach einem der vorhergehenden Ansprüche und mindestens eine selbstklebende Platte (50), die dazu geeignet ist, zwischen das genannte Kompressionskleidungsstück und einen Bereich des Körpers des Patienten eingefügt zu werden.

9. Satz umfassend ein Kompressionskleidungsstück nach einem der vorhergehenden Ansprüche und mindestens ein Kompressionskissen (60) aus Schaumstoff, das dazu geeignet ist, zwischen das genannte Kompressionskleidungsstück und einen Bereich des Körpers des Patienten eingefügt zu werden.

10. Verfahren zur Herstellung eines Kompressionskleidungsstücks eines Körperteils eines Patienten, umfassend :
• die Bereitstellung von mindestens zwei Trägerbändern, die jeweils mindestens eine jeweilige Silikonbahn (1) aufweisen, und
• die Anordnung der genannten mindestens zwei Trägerbänder auf einer Innenseite eines Kleidungsstücks aus einem kompressiven Textilstoff, der mindestens zwei Zonen umfasst, die durch elastische Nähte (20) zusammengefügt sind, wobei der Nahtüberschuss an der Außenseite des Kleidungsstücks angeordnet ist, so dass die Silikonbahnen zur Hautseite des Patienten orientiert sind, wobei die Trägerbänder einen gemeinsamen Punkt in einem oberen Abschnitt des Kleidungsstücks aufweisen und der Abstand zwischen den Trägerbändern mit zunehmender Entfernung vom gemeinsamen Punkt entlang mindestens eines Lymphdrainagewegs des genannten Körperteils des Patienten zunimmt.

## Claims

1. Compression garment for postoperative use suitable for ensuring a compression of a part of the body of a patient, **characterized in that** it comprises :
• at least one compressive textile comprising at least two zones assembled by elastic seams (20) whose seam excess is arranged on the outside of the garment, and
• a plurality of silicone tracks (1) arranged on a plurality of support bands arranged on a face of the compressive textile intended to be in contact with the skin of the patient, said support bands having a common point in an upper portion of the garment, the distance between the support bands increasing at an increasing distance from the common point so that the silicone tracks adhere to the skin of the patient following a respective lymphatic drainage path of said part of the body of the patient.

2. Compression garment according to claim 1, in which the silicone tracks (1) form a relief on the support band.

3. Compression garment according to one of the preceding claims, in which the silicone tracks (1) are wavy, preferably sinusoidal.

4. Compression garment according to one of the preceding claims, in which each support band comprises several parallel silicone tracks, preferably two parallel silicone tracks.

5. Compression garment according to one of the preceding claims, presented in the form of an abdominal compression girdle.

6. Compression garment according to one of claims 1 to 5, presented in the form of a compression garment of the lower limbs (210).

7. Compression garment according to one of claims 1 to 5, presented in the form of a breast compression garment (310).

8. Set comprising a compression garment according to one of the preceding claims and at least one self-adhesive plate (50) suitable for being inserted between said compression garment and an area of the body of the patient.

9. Set comprising a compression garment according to one of the preceding claims and at least one compression cushion (60) made of foam suitable for being inserted between said compression garment and an area of the body of the patient.

10. Method for manufacturing a compression garment of a part of the body of a patient, comprising :
• providing of at least two support bands comprising at least one respective silicone track (1), and
• arranging of said at least two support bands on an inner face of a garment made of a compressive textile comprising at least two zones assembled by elastic seams (20) whose seam excess is arranged on the outside of the garment, so that the silicone tracks are oriented towards the side of the skin of the patient, the support bands having a common point in an upper portion of the garment, the distance between the support bands increasing at an increasing distance from the common point following at least one lymphatic drainage path of said part of the body of the patient.
